# EUROPÄISCHE PATENTSCHRIFT

(11) **EP 0 796 111 B1**
(45) Veröffentlichungstag und Bekanntmachung des Hinweises auf die Patenterteilung: **23.04.2003**
(21) Anmeldenummer: 95935348.3
(22) Anmeldetag: 10.10.1995
(51) Int. Cl.: A61K 49/00

(54) **VERFAHREN ZUR IN-VIVO-DIAGNOSTIK MITTELS NIR-STRAHLUNG UNTER VERWENDUNG VON CYANINFARBSTOFFEN**
IN-VIVO DIAGNOSTIC PROCESS BY NEAR INFRARED RADIATION USING CYANINE DYES
PROCEDE DE DIAGNOSTIQUE IN VIVO PAR RAYONS INFRAROUGES PROCHES A L'AIDE DE COLORANTS DE CYANINE

(30) Priorität: 07.12.1994 DE 4445065
(43) Veröffentlichungstag der Anmeldung: 24.09.1997
(60) Teilanmeldung: 01250366.0
(73) Patentinhaber: Institut für Diagnostikforschung GmbH an der Freien Universität Berlin, 13353 Berlin (DE)
(72) Erfinder: LICHA, Kai, D-14169 Berlin (DE); RIEFKE, Björn, D-13595 Berlin (DE); SEMMLER, Wolfhard, D-16548 Glienicke (DE); SPECK, Ulrich, D-13465 Berlin (DE); HILGER, Christoph-Stephan, D-13353 Berlin (DE)
(74) Vertreter: Seuss, Thomas
(86) Internationale Anmeldenummer: DE9501465
(87) Internationale Veröffentlichungsnummer: WO96017628

(56) Entgegenhaltungen:
- WO-A-89/10758
- WO-A-91/18006
- WO-A-92/00748
- DE-A- 4 136 769
- DE-A- 4 323 368
- J. FLUORESC. (1993), 3(3), 153-5 CODEN: JOFLEN;ISSN: 1053-0509, 1993 TERPETSCHNIG, EWALD ET AL 'An investigation of squaraines as a new class of fluorophores with long-wavelength excitation and emission'
- ANALYTICAL CHEMISTRY, 1993 COLUMBUS US, Seiten 1742-1748, TERPETSCHNIG, EWALD ET AL 'Synthesis, spectral properties and photostabilities of symmetrical and unsymmetrical squaraines;a new class of fluorophores with long-wavelength excitation and emission'
- BIOCONJUGATE CHEMISTRY, Bd. 4, Nr. 2, April 1993 WASHINGTON US, Seiten 105-111, RATNAKAR B. MUJUMDAR ET AL. 'CYANINE DYE LABELING RAEGENTS: SULFOINDOCYANINE SUCCINIMIDYL ESTERS.' in der Anmeldung erwähnt
- CANCER RESEARCH, Bd. 54, 15.Mai 1994 MD US, Seiten 2643-2649, SILVIO FOLLI ET AL. 'ANTIBODY-INDOCYANIN CONJUGATES FOR IMMUNOPHOTODETECTION OF HUMAN SQUAMOUS CELL CARCINOMA IN NUDE MICE.' in der Anmeldung erwähnt
- ANAL. CHIM. ACTA (1993), 282(3), 633-41 CODEN: ACACAM;ISSN: 0003-2670, 1993 TERPETSCHNIG, EWALD ET AL 'Synthesis, spectral properties and photostabilities of symmetrical and unsymmetrical squaraines;a new class of fluorophores with long-wavelength excitation and emission'

## Beschreibung

Die Erfindung betrifft die Verwendung von Cyaninfarbstoffen zur Herstellung von Mitteln für ein Verfahren zur In-vivo-Diagnostik mittels Nahinfrarot-Strahlung (NIR-Strahlung) unter Verwendung von wasserlöslichen Farbstoffen mit bestimmten photophysikalischen und pharmakochemischen Eigenschaften als Kontrastmittel in der Fluoreszenz- und Transilluminationsdiagnostik im NIR-Bereich, neue Farbstoffe und diese enthaltende pharmazeutische Mittel.

Die Erkennung von Krankheiten ist zu einem wesentlichen Teil davon abhängig, inwieweit es gelingt, Informationen über Strukturen und deren Veränderungen aus den primär nicht zugänglichen tieferen Schichten der Gewebe zu erlangen. Dies kann neben Tasten, Freilegen oder Punktieren durch die modernen bildgebenden Verfahren, wie Röntgen, die Magnetresonanztomographie oder die Ultraschalldiagnostik geschehen.

Da biologisches Gewebe eine relativ hohe Durchlässigkeit für langwelliges Licht des Wellenlängenbereiches von 650 - 1000 nm besitzt, steht dem Diagnostiker hiermit ein völlig anderes Verfahren zur bildlichen Gewebedarstellung zur Verfügung. Die Tatsache, daß nahinfrarotes Licht Gewebe bis zu mehreren Zentimetern durchdringen kann, wird in der Transilluminationsbildgebung genutzt. Diese Technik erlaubt bisher die Diagnose von Entzündungen der Nasennebenhöhlen und Kiefernhöhlen oder den Nachweis von Flüssigkeitsansammlungen oder Blut in oberflächennahen Geweberegionen (Beuthan J., Müller G.; Infrarotdiaphanoskopie, Med. Tech. **1** (1992) 13-17).

Versuche zur Erkennung von Brusttumoren verliefen bisher unbefriedigend (Navarro, G.A.; Profio, A.E.; Contrast in diaphanography of the breast; Med. Phys. **150** (1988) 181-187; Aspegren, K.; Light Scanning Versus Mammography for the Detection of Breast Cancer in Screening and Clinical Practice, Cancer 65 (1990) 1671-77), versprechen aber aufgrund neuester gerätetechnischer Entwicklungen besseren Erfolg (Klingenbeck J.; Laser-Mammography with NIR-Light, Gynäkol.-Geburtsh.-Rundsch. **33** Suppl.1 (1993) 299-300); Benaron D.A.; Optical Imaging reborn with technical advances, Diagnostic Imaging (1994) 69-76).

Neben der Detektion der nicht absorbierten Strahlung kann auch die nach Bestrahlung mit nahinfrarotem Licht emittierte Fluoreszenzstrahlung gewebespezifische Informationen liefern. Diese sogenannte Autofluoreszenz wird genutzt, um atherosklerotisches von normalem Gewebe zu unterscheiden (Henry, P. D. et al., Laser-Induced Autofluorescence of Human Arteries, Circ. Res. **63** (1988) 1053-59).

Das wesentliche Problem bei der Nutzung von nahinfraroter Strahlung ist die außerordentlich starke Streuung des Lichtes, so daß selbst bei unterschiedlichen photophysikalischen Eigenschaften sich ein scharf begrenztes Objekt von seiner Umgebung nur unscharf abzeichnet. Das Problem nimmt mit wachsender Entfernung von der Oberfläche zu und kann als hauptsächlicher limitierender Faktor sowohl bei der Transillumination als auch bei der Detektion von Fluoreszenzstrahlung angesehen werden.

Zur Verbesserung der Differenzierung zwischen normalem und erkranktem Gewebe können geeignete Fluoreszenzfarbstoffe beitragen, die sich im erkrankten Gewebe (insbesondere Tumoren) anreichern und ein spezifisches Absorptions- und Emissionsverhalten besitzen. Die durch Absorption des Farbstoffes bewirkte Änderung des (gestreuten) eingestrahlten Lichtes oder die durch die Anregerstrahlung induzierte Fluoreszenz wird detektiert und liefert die eigentlichen gewebespezifischen Informationen.

Beispiele für die Anwendung von Farbstoffen für die In-vivo-Diagnostik beim Menschen sind die Verfolgung im Blut mit photometrischen Methoden zur Erkennung von Verteilungsräumen, Blutfluß oder Stoffwechsel- und Ausscheidungsfunktionen, sowie die Sichtbarmachung durchsichtiger Strukturen des Auges (Ophthalmologie). Bevorzugte Farbstoffe für diese Anwendungen sind das Indocyaningrün und das Fluorescein (Googe, J.M. et al., Intraoperative Fluorescein Angiography; Ophthalmology, **100**, (1993), 1167-70).

Indocyanin-Grün (Cardiogreen) wird zur Messung von Leberfunktion, Herzzeit- und Schlagvolumen, sowie von Organ- und peripherer Durchblutungen verwendet (I. Med. 24 (1993)10-27) und als Kontrastmittel zur Tumordetektion erprobt. Indocyanin-Grün bindet zu 100% an Albumin und wird in der Leber freigesetzt. Die Fluoreszenzquantenausbeute ist in wäßrigem Milieu gering. Die LD₅₀ (0,84 mmol/kg) ist ausreichend hoch, es können jedoch starke anaphylaktische Reaktionen hervorgerufen werden. Indocyanin-Grün ist in Lösung instabil und kann nicht in salzhaltigen Medien appliziert werden, da es zur Ausfällung kommt.

Für die Lokalisation und Abbildung von Tumoren wurden bisher die für eine Anwendung in der Photodynamischen Therapie (PDT) konzipierten Photosensibilisatoren (u.a. Hämatoporphyrinderivate, Photophrin II, Benzoporphyrine, Tetraphenylporphyrine, Chlorine, Phthalocyanine) verwendet (Bonnett R.; New photosensitizers for the photodynamic therapy of tumours, SPIE Vol. 2078 (1994)). Die aufgeführten Verbindungen haben den gemeinsamen Nachteil, daß sie im Wellenlängenbereich von 650 - 1200 nm nur eine mäßige Absorption aufweisen. Die für die PDT erforderliche Phototoxizität ist für rein diagnostische Zielstellungen störend. Weitere Schriften zu dieser Thematik: U.S.-PS 4945239; WO 84/04665, WO 90/10219, DE-OS 4136769, DE-PS 2910760.

In der U.S.-PS 4945239 werden zahlreiche apparative Anordnungen zur Detektion von Brustkrebs mittels Transillumination beschrieben und als kontrasterhöhende Absorptionsfarbstoffe das bekannte Fluorescein, Fluorescamin und Riboflavin genannt. Diese Farbstoffe haben den gemeinsamen Nachteil, daß sie im sichtbaren Wellenlängenbereich von 400-600 nm, in welchem die Lichtdurchlässigkeit von Gewebe sehr gering ist, absorbieren.

In der DE-OS 4136769 ist eine Apparatur zur Fluoreszenzdetektion von mit fluoreszierenden Substanzen angereicherten Gewebebereichen beschrieben. Die Substanzen sind Bacteriochlorophyll und Derivate, sowie Naphthalocyanine. Diese Strukturen zeichnen sich durch Absorptionen im Bereich von 700-800 nm mit Extinktionskoeffizienten von bis zu 70000 1 mol⁻¹ cm⁻¹ aus. Die hier aufgeführten Verbindungen besitzen neben den Fluoreszenzeigenschaften die Fähigkeit, durch Bestrahlung Singulettsauerstoff zu generieren und dadurch zytotoxisch zu wirken (Photosensibilatoren für die photodynamische Therapie). Diese photosensibilisierende Wirkung ist für ein reines, wirkungsfreies Diagnostikum in höchstem Maße unerwünscht.

Darüberhinaus ist die Synthese der Bacteriochlorophyllverbindungen zudem aufwendig und teuer, da Naturprodukte als Ausgangssubstanzen erforderlich sind; die Naphthalocyanine zeichnen sich oft durch eine sehr geringe Photostabilität aus. Die bekannten Verbindungen dieser Klassen sind schlecht wasserlöslich, die Synthese einheitlicher, hydrophiler Derivate ist aufwendig.

WO 84/04665 beschreibt ein In-vivo-Verfahren zur Fluoreszenzdetektion von Tumoren unter Verwendung der Photosensibilisatoren Hämatoporphyrin und -derivat (Hp und HpD), Uro- und Copro- und Protoporphyrin und zahlreicher mesosubstituierter Porphyrine, sowie der Farbstoffe Riboflavin, Fluorescein, Acridin-Orange, Berberinsulfat und von Tetracyclinen. Die oben genannten photophysikalischen und pharmakochemischen Anforderungen werden von den genannten Substanzen nicht erfüllt.

Folli et al., Cancer Research 54, 2643-2649 (1994), beschreiben einen mit einem Cyaninfarbstoff verbundenen monoklonalen Antikörper, der zum Nachweis eines subkutan implantierten Tumors verwendet wurde. Der Nachweis tiefer gelegener pathologischer Prozesse macht allerdings die Verwendung weiter verbesserter Farbstoffe erforderlich. Ferner lassen höhere Farbstoffdosierungen die Verwendung von Antikörpern als Träger aufgrund zu erwartender Nebenwirkungen als ungeeignet erscheinen.

Der in Folli et al. zur Herstellung der Antikörper-Konjugate verwendete Farbstoff wird in Terpetschnig et al., Journal of Fluorescence, Vol. 3, No. 3, 1993, S. 153ff., beschrieben. Die Farbstoffe werden in in-vitro-Verfahren, nämlich Fluoreszenzassays, eingesetzt.

Terpetschnig et al., Analytica Chimica Acta, Vol. 282 (1993), S. 633 - 641, beschreibt die Synthese der Squarainfarbstoffe, welche im obengenannten Artikel aus Journal of Fluorescence beschrieben sind.

Mujumdar et al., Bioconjugate Chemistry, Vol. 4, No. 2, 1993, S. 105ff., beschreibt wasserlösliche Cyaninfarbstoffe, welche als Fluoreszenzmarker eingesetzt werden.

WO 89/10758 beschreibt Fluoreszenzverbindungen mit lipophilen Resten. Die Verbindungen sind schlecht wasserlöslich, aber gut fettlöslich, damit sie in die Zellmembran eingebaut werden können und dort verbleiben.

WO 91/18006 beschreibt fluoreszierende Porphyrine und Phthalocyanine.

Cyanin-Farbstoffe und damit verwandte Polymethinfarbstoffe finden auch als Bestandteile photographischer Schichten Verwendung. Solche Farbstoffe benötigen keine Lumineszenzeigenschaften. Cyaninfarbstoffe mit Lumineszenz-(Fluoreszenz-)eigenschaften sind für die Verwendung in der Fluoreszenzmikroskopie und Durchflußzytometry synthetisiert und an Biomoleküle gekoppelt worden, z. B. Verbindungen mit Iodacetylgruppen als spezifische Marker für Sulfhydrylgruppen von Proteinen (Waggoner, A.S. et al.; Cyanine dye Labeling Reagents for Sulfhydryl Groups, Cytometry, **10**, (1989), 3-10). Auf diese Weise werden Proteine markiert und isoliert. Weitere Literaturbeispiele: Cytometry 12 (1990) 723-30; Anal. Lett. 25 (1992) 415-28; Bioconjugate Chem. 4 (1993) 105-11.

In der DE-OS 39 12 046 von Waggoner, A.S. wird ein Verfahren zur Markierung von Biomolekülen mit Cyanin- und verwandten, wie Merocyanin- und Styrylfarbstoffen, die mindestens eine Sulfonat- oder Sulfonsäuregruppierung enthalten, beschrieben. Die erwähnte Schrift betrifft ein ein- sowie zweistufiges Markierungsverfahren in einem wäßrigen Medium, wobei eine kovalente Reaktion zwischen Farbstoff und Amin-, Hydroxy-, Aldehyd- oder Sulfhydrylgruppe auf Proteinen oder anderen Biomolekülen stattfindet.

DE-OS 3828360 betrifft ein Verfahren zur Markierung von Antitumor-Antikörpern, speziell Melanom- und Kolonkarzinomspezificher Antikörper, mit Fluorescein und Indocyanin-Grün für ophthalmologische Zielstellungen. Die Bindung von Indocyanin-Grün an Biomoleküle ist nicht kovalent (Farbstoff-Antikörperkombination, Mischung).

Die aus dem Stand der Technik bekannten Verfahren zur In-vivo-Diagnostik mittels NIR-Strahlung weisen somit eine Reihe von Nachteilen auf, durch welche deren Anwendung in der breiten medizinischen Diagnostik bisher unmöglich war.

Die direkte Verwendung von sichtbarem Licht oder NIR-Strahlung ist auf oberflächennahe Körperbereiche beschränkt, was mit der starken Streuung des eingestrahlten Lichtes zusammenhängt.

Der Zusatz von Farbstoffen zur Verbesserung des Kontrastes und des Auflösungsvermögens ruft jedoch eine Reihe weiterer Probleme hervor. Es sind nämlich an diese Farbstoffe Maßstäbe anzulegen, welche allgemein für Diagnostika gelten. So dürfen solche Stoffe, da diese meist in höheren Dosen, auch über einen längeren Diagnosezeitraum verabfolgt werden, nur eine geringe Toxizität aufweisen. Ferner müssen diese für die Diagnose geeigneten Farbstoffe gut wasserlöslich und hinreichend, d. h. mindestens während der Diagnosedauer, chemisch und photophysikalisch stabil sein. Auch ist eine Stabilität bezüglich der Metabolisierung im Organismus anzustreben.

Bisher stehen weder Farbstoffe mit solchen Eigenschaften noch ein geeignetes Verfahren zur In-vivo-Diagnostik mittels NIR-Strahlung zur Verfügung.

Der Erfindung liegt daher die Aufgabe zugrunde, ein Verfahren zur In-vivo-Diagnostik zu schaffen, welches die Nachteile des Standes der Technik überwindet.

Diese Aufgabe wird erfindungsgemäß dadurch gelöst, daß Cyaninfarbstoffe zur Herstellung von Mitteln für ein Verfahren zur In-vivo-Diagnostik mittels NIR-Strahlung, zur Verfügung gestellt wird, werden, wobei Verbindungen gemäß Anspruch 1-4 verwendet werden.

Die erfindungsgemäße verwendeten Verbindungen zeichnen sich dadurch aus, daß sie im Wellenlängenbereich von 650 bis 1200 nm absorbieren und fluoreszieren, Absorptionskoeffizienten von ca. 100 000 1 mol⁻¹ cm⁻¹ und höher und, soweit Fluoreszenz erwünscht ist, Fluoreszenzquantenausbeuten größer 5%, eine ausreichende Wasserlöslichkeit, Verträglichkeit und In-vitro- und In-vivo- sowie Photostabilität besitzen. Sie werden in möglichst kurzer Zeit möglichst vollständig wieder ausgeschieden. Die erfindungsgemäß verwendeten Verbindungen sind leicht synthetisch und preisgünstig in möglichst wenigen Reaktionsstufen aus käuflich erhältlichen Ausgangsmaterialien zugänglich.

Den Geweben wird bei der Durchführung des Verfahrens zur In-vivo-Diagnostik eine oder mehrere der Substanzen gemäß der allgemeinen Formel V z. B. durch intravenöse Injektion zugeführt und Licht aus dem sichtbaren bis nahinfraroten Bereich von 650 bis 1200 nm eingestrahlt. Die nicht absorbierte Strahlung und die Fluoreszenzstrahlung wird einzeln oder beide gleichzeitig oder beide gegeneinander zeitverzögert registriert. Aus den erhaltenen Daten wird ein synthetisches Bild erzeugt.

Die Aufnahme von Fluoreszenzbildern kann nach unterschiedlichen Methoden erfolgen. Bevorzugt sind die Methoden, bei denen das Gewebe großflächig bestrahlt und die Fluoreszenz-Information örtlich aufgelöst durch Aufnahme mit einer CCD-Kamera dargestellt wird oder die abzubildenden Gewebeareale mit einem in einen optischen Lichtleiter eingebündelten Lichtstrahl abgetastet und die erhaltenen Signale rechnerisch in ein Bild umgesetzt werden. Das Licht wird schmalbandig im Bereich der Absorptionsmaxima bzw. Fluoreszenzanregungswellenlängen der erfindungsgemäßen Verbindungen eingestrahlt. Die nicht absorbierte Strahlung kann ebenfalls in der beschriebenen Weise detektiert und die erhaltenen Signale verarbeitet werden.

Einstrahl- und Beobachtungswinkel sind nach den anatomischen Gegebenheiten und dem optimalen Kontrast von Fall zu Fall wählbar. Durch Subtraktion der Bilder vor und nach der Farbstoffgabe ist die Empfindlichkeit der Methode weiter zu verbessern. Durch Auswertung des Zeitverlaufs der farbstoffbedingten Veränderungen können für die Diagnostik nützliche Zusatzinformationen gewonnen werden.

Die meßtechnischen Verfahren sind dem Fachmann geläufig. Dem Fachmann ist ferner bekannt, welche apparativen Parameter zu wählen sind, um bei vorgegebener Absorptions bzw. Fluoreszenzwellenlänge der erfindungsgemäße verwendeten Farbstoffe der allgemeinen Formel I, optimale Aufzeichnungs- und Auswertungsbedingungen zu erzielen.

Die erfindungsgemäß verwendeten Verbindungen der allgemeinen Formel V decken durch die variable strukturelle Natur des Farbstoffsystems F einen weiten Anregungs- und Emissionswellenlängenbereich ab. Es besteht die Möglichkeit, Produkte mit Anregungswellenlängen zu erhalten, die einer besonderen Anregungsquelle, z. B. einem Diodenlaser, entsprechen und somit an eine vorgegebene Meßapparatur bzw. gerätetechnische Komponente angepaßt sind.

Mittels der beschriebenen Techniken lassen sich selbst kleine Objekte von wenigen mm³ Volumen in tieferen Schichten der Gewebe oder in nicht transparenten Körperflüssigkeiten orten. Es bleibt aufgrund der Lichtstreuung und der damit verbundenen begrenzten Auflösung schwierig, die genaue Form und Größe der Objekte zu bestimmen, was für einige wichtige diagnostische Fragestellungen auch nicht erforderlich ist.

Überraschenderweise ergab eine nach Applikation eines Cyaninfarbstoffes durchgeführte Fluoreszenzlichtaufnahme einer Nacktmaus mit einer CCD-Kamera eine Fluoreszenzintensität, die um das tausendfache höher war, als bei einem entsprechend dosierten Porphyrin.

Das beschriebene Verfahren unter Verwendung der erfindungsgemäßen Verbindungen eignet sich besonders zur bildlichen Darstellung von nicht pathologisch verändertem Gewebe, systemischen Erkrankungen, Tumoren, Blutgefäßen, artherosklerotischen Plaques und der Perfusion und Diffusion.

Die erfindungsgemäß verwendeten Verbindungen werden auf unterschiedliche Weise in das Gewebe eingebracht. Besonders bevorzugt ist die intravenöse Gabe der Farbstoffe.

Die Dosierung kann entsprechend dem Zweck der Anwendung sehr unterschiedlich sein. Ziel ist eine erkennbare Farbstoffkonzentration im zu diagnostizierenden Gewebebereich, wozu meist eine Konzentration von 1-100 µg/ml im Gewebe oder in Körperflüssigkeiten ausreichend ist. Dieses Ziel wir bei direkter Injektion in kleine Körperhöhlen oder kleine Blut- oder Lymphgefäße im allgemeinen durch Verabreichung von 0,1-100 mg des betreffenden Farbstoffes enthalten in 0,1 bis 10 ml Trägerflüssigkeit erreicht. Bevorzugt sind in diesem Falle 1 bis 10 mg Farbstoff. Zur Anfärbung der Blutgefäße oder zur Erkennung spezieller Gewebe oder Strukturen nach intravenöser Injektion sind meist höhere Dosierungen (größer gleich 100 mg) erforderlich. Die obere Grenze der Dosierung ist nur durch die Verträglichkeit der jeweiligen Substanzen und Zubereitungen gegeben.

Die Erfindung betrifft Verbindungen der Ansprüche 1-4 deren n-Octanol/Wasser-Verteilungskoeffizient kleiner 2 ist (n-Octanol/0,01 M TRIS-Puffer mit 0,9 % Kochsalz, auf pH 7,4 eingestellt, beide Phasen gegeneinander gesättigt).

In den erfindungsgemäß verwendeten Verbindungen der allgemeinen Formel V bedeutet Alkyl-, Aryl- oder Aralkylrest mit Hydroxygruppen beispielsweise 2-Hydroxyethyl-, 2-Hydroxypropyl-, 3-Hydroxypropyl-, 4-Hydroxybutyl-, 2,3-Dihydroxypropyl-, 1,3-Dihydroxyprop-2-yl-, Tris-(Hydroxymethyl)-methyl-, 1,3,4-Trihydroxybut-2-yl-Glucosyl-, 4-(1,2-Dihydroxyethyl)phenyl- oder 2,4-, 2,5-, 3,5- oder 3,4-Dihydroxyphenyl-Reste.

Ein Alkyl-, Aryl- oder Aralkylrest mit 1 - 3 Carboxygruppen kann beispielsweise ein Carboxymethyl-, Carboxyethyl-, Carboxypropyl-, Carboxybutyl-, 1,2-Dicarboxyethyl-, 1,3-Dicarboxypropyl-, 3,5-Dicarboxyphenyl-, 3,4-Dicarboxyphenyl-, 2,4-Dicarboxyphenyl oder 4-(1,2-Dicarboxyehtyl)-phenyl-Rest sein.

Unter einem Sulfoalkylrest ist bevorzugt ein 2-Sulfoethyl-, 3-Sulfopropyl- und ein 4-Sulfobutylrest zu verstehen.

Die erfindungsgemäß verwendeten Farbstoffe absorbieren im Spektralbereich von 650 nm bis 1200 nm. Die Absorptionskoeffizienten der Verbindungen sind ca. 100000 1 mol⁻¹ cm⁻¹ und größer bezogen auf ein Farbstoffmolekül. Die Fluoreszenzquantenausbeuten sind größer als 5% bei Farbstoffen, die für die Fluoreszenzbildgebung verwendet werden.

Gegenstand der vorliegenden Erfindung sind Cyaninfarbstoffe der allgemeinen Formel V. 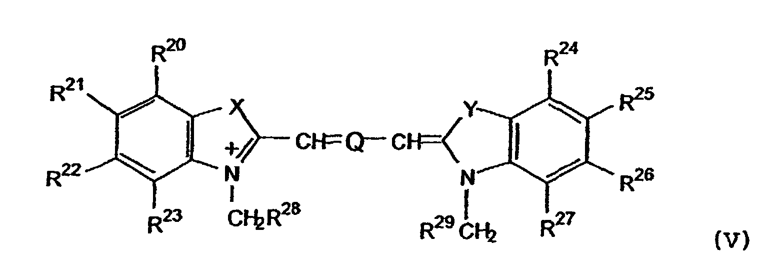 wobei
Q ein Fragment 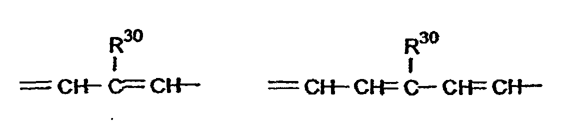 oder 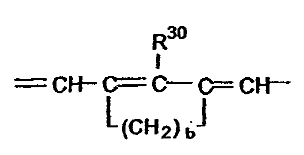 ist,
wobei R³⁰ für ein Wasserstoffatom, eine Hydroxygruppe, eine Carboxygruppe, einen Alkoxyrest mit 1 bis 4 Kohlenstoffatomen oder ein Chloratom steht, b eine ganze Zahl 2 oder 3 bedeutet,
X und Y unabhängig voneinander für ein Fragment -O-, -S-, -CH=CH- oder -C(CH₂R³²)(CH₂R³³)- stehen, R²⁰ bis R²⁹, R³² und R³³ unabhängig voneinander für ein Wasserstoffatom, eine Hydroxygruppe, einen Carboxy-, einen Sulfonsäure-Rest oder einen Carboxyalkyl-, Alkoxycarbonyl oder Alkoxyoxoalkyl-Rest mit bis zu 10 C-Atomen oder ein Sulfoalkylrest mit bis zu 4 C-Atomen
oder für ein Polylysin,Polyethylenglycol,Methoxypolyethylenglycol, Polyvinylalkohol, Dextran, Carboxydextran oder eine kaskadenpolymerartige Struktur, oder für einen Rest der allgemeinen Formel VI

-**(O)**_{**v**}**-(CH**_{**2**}**)**_{**o**}**-CO-NR**^{**34**}**-(CH**_{**2**}**)**_{**s**}**-(NH-CO)**_{**q**}**-R**^{**35**} (VI)

steht,
mit der Maßgabe, daß bei der Bedeutung von X und Y gleich O, S, -CH=CH- oder -C(CH₃)₂- mindestens einer der Reste R²⁰ bis R²⁹ einem Polylysin, Polyethylenglycol, Methoxypolyethylenglycol, Polyvinylalkohol, Dextran, Carboxydextran oder einer kaskadenpolymerartigen Struktur, oder für einen Rest der allgemeinen Formel VI entspricht,
wobei
o und s gleich 0 sind oder unabhängig voneinander für eine ganze Zahl von 1 bis 6 stehen, q und v unabhängig voneinander für 0 oder 1 stehen,
R³⁴ ein Wasserstoffatom oder einen Methylrest darstellt,
R³⁵ ein Alkylrest mit 3 bis 6 C-Atomen, welcher 2 bis n-1 Hydroxygruppen aufweist, wobei n die Anzahl der C-Atome ist, oder ein mit 1 bis 3 Carboxygruppen substituierter Alkylrest mit 1 bis 6 C-Atomen, Arylrest mit 6 bis 9 C-Atomen oder Arylalkylrest mit 7 bis 15 C-Atomen, oder ein Rest der allgemeinen Formel IIId oder IIIe 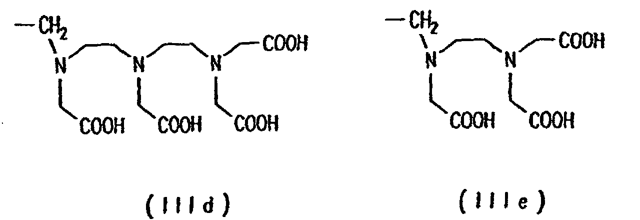 ist,
unter der Maßgabe, daß q für 1 steht, oder ein Polylysin,Polyethylenglycol, Methoxypolyethylenglycol Polyvinylalkohol, Dextran, Carboxydextran oder eine kaskadenpolymerartige Struktur bedeutet,
oder R²⁰ und R²¹, R²¹ und R²², R²² und R²³, R²⁴ und R²⁵, R²⁵ und R²⁶ oder R²⁶ und R²⁷ zusammen mit den zwischen ihnen liegenden Kohlenstoffatomen einen 5- oder 6-gliedrigen aromatischen oder gesättigten annelierten Ring bilden, sowie deren physiologisch verträgliche Salze, wobei die Cyaninfarbstoffe eine durch einen n-Octanol/Wasser-Verteilungskoeffizienten von kleiner 2,0 gekennzeichnete Hydrophilie besitzen.

In den erfindungsgemäßen Verbindungen der allgemeinen Formel V haben die Alkyl-, Aryl- oder Aralkylreste mit Hydroxy- oder Carboxygruppen die zuvor als bevorzugt erwähnten Bedeutungen.

Besonders bevorzugte Cyaninfarbstoffe sind
5-[2-[(1,2-Dicarboxyethyl) amino]-2-oxoethyl]-2-[7-[5-[2-(1,2-dicarboxyethyl)amino]-2-oxoethyl]-1,3-dihydro-3,3-dimethyl-1-(4-sulfobutyl)-2*H*-indol-2-yliden]-1,3,5-heptatrienyl]-3,3-dimethyl-1-(4-sulfobutyl)-3*H*-indolium, inneres Salz, Monokaliumsalz,
2-[7-[5-[2-[(11-Carboxy-2-oxo-1,4,7,10-tetraaza-4,7,10-tri(carboxymethyl)-1-undecyl)amino]-2-oxoethyl]-1,3-dihydro-3,3-dimethyl-1-ethyl-2*H*-indol-2-yliden]-1,3,5-heptatrienyl]-3,3-dimethyl-1-(4-sulfobutyl)-3H-indolium, inneres Salz,
2-[7-[1,3-Dihydro-3,3-dimethyl-5-[2-[(Methoxypolyoxyethylen)-amino] -2-oxoethyl]-1-(4-sulfobutyl)-2Hindol-2-yliden]-1,3,5-heptatrienyl]-3,3-dimethyl-5-[2-[(methoxypolyoxyethylen)amino]-2-oxoethyl]-1-(4-sulfobutyl)-3H-indolium, Natriumsalz,
2-[7-[1,3-Dihydro-3,3-dimethyl-1-(4-sulfobutyl) -2Hindol-2-yliden]-1,3,5-heptatrienyl]-3,3-dimethyl-5-(Methoxypolyoxyethylen)aminocarbonyl-1-(4-sulfobutyl)-3H-indolium, Natriumsalz,
3-(3-Carboxypropyl)-2-[7-[3-(3-carboxypropyl)-1,3-dihydro-3-methyl-1-(4-sulfobutyl)-2*H*-indol-2-yliden]-1,3,5-heptatrienyl]-3-methyl-1-(4-sulfobutyl)-3*H*indolium, Natriumsalz,
2-[7-[1,3-Dihydro-5-[2-[(2,3-dihydroxypropyl)amino]-2-oxoethyl]-3,3-dimethyl-1-(4-sulfobutyl)-2H-indol-2-yliden]-1,3,5-heptatrienyl]-5-[2-[(2,3-dihydroxypropyl)amino]-2-oxoethyl]-3,3-dimethyl-1-(4-sulfobutyl)-3H-indolium, Natriumsalz

Eine wesentliche Eigenschaft der erfindungsgemäßen Verbindungen und der erfindungsgemäß verwendeten Verbindungen besteht darin, daß sie eine durch einen n-Octanol/Wasser-Verteilungskoeffizienten von kleiner 2,0 gekennzeichnete Hydrophilie besitzen (Verteilungskoeffizient n-Octanol / 0,01 M TRIS-Puffer mit 0,9 % Kochsalz, auf pH 7,4 eingestellt, beide Phasen gegeneinander gesättigt). Die Verbindungen besitzen keine ausgeprägten, für ein reines Diagnostikum unerwünschten photosensibilisierenden bzw. phototoxischen Eigenschaften. Sie sind weiterhin gut verträglich und werden ausgeschieden.

Durch diese Eigenschaft grenzen sich die Verbindungen von den bisher bekannten, für die In-vivo-Diagnostik vorgeschlagenen oder verwendeten Farbstoffen ab. Die Fluoreszenzquantenausbeuten, die besonders bei Cyaninfarbstoffen in wäßrigen Medien durch Aggregation drastisch absinken, sind vergleichbar mit den in apolaren Lösungsmitteln gemessenen, da durch die erhöhte Wasserlöslichkeit und den Raumanspruch der hydrophilen Gruppen eine Aggregat- und Micellenbildung unterdrückt wird.

Die Proteinaffinität einer Gruppe der bevorzugten Verbindungen ist gering, das pharmakokinetische Verhalten entspricht in etwa dem von beispielsweise Insulin oder Saccharose.

Überraschenderweise zeigte sich, daß auch bei diesen Verbindungen trotz des einfachen Molekülaufbaus eine diagnostisch ausreichende Anreichung in bestimmten Strukturen im Organismus, z.B. auch in Tumoren erfolgt und nach Gleichverteilung des Farbstoffes im Organismus die Elimination aus den Tumorbereichen im Vergleich zum umliegenden Gewebe verzögert stattfindet.

Die Verträglichkeit der Substanzen ist sehr hoch. Besonders bevorzugt sind Substanzen mit LD₅₀-Werten größer 0,5 mmol/kg Körpergewicht bezogen auf ein einzelnes Farbstoffmolekül.

Die erfindungsgemäßen und erfindungsgemäß verwendeten Verbindungen zeichnen sich durch eine hohe In-vitro- und In-vivo-, sowie Photostabilität aus. Bei Stehenlassen einer wäßrigen Lösung in tagesbelichteten Räumen sind bei den besonders bevorzugten Verbindungen nach 2 Tagen 98 % und nach 12 Tagen 70 % unverändert.

Die beschriebenen photophysikalischen und pharmakokinetischen Eigenschaften der erfindungsgemäßen und erfindungsgemäß verwendeten Verbindungen unterscheiden sich auch von denen des einzigen zur Anwendung am Patienten zugelassenen Cyaninfarbstoffes, dem Indocyanin-Grün (Cardiogreen).

Es ist die Synthese von Cyaninfarbstoffen möglich, die bei Wellenlängen von 650 bis 1200 nm mit hohen Extinktionskoeffizienten absorbieren und in hoher Effizienz fluoreszieren. Die Synthese der erfindungsgemäßen und erfindungsgemäß verwendeten Cyaninfarbstoffe erfolgt überwiegend in Anlehnung an literaturbekannte Methoden, beispielsweise F.M. Hamer in The Cyanine Dyes and Related Compounds, John Wiley and Sons, New York, 1964; Cytometry, **10** (1989) 3-10; **11** (1990) 418-430; **12** (1990) 723-30; Bioconjugate Chem. 4 (1993) 105-11, Anal. Biochem. **217** (1994) 197-204; Tetrahedron **45** (1989) 4845-66, European Patent Appl. 0 591 820 A1.

Die Substanzen können sehr unterschiedliche Eigenschaften haben. Das Molekulargewicht kann zwischen wenigen Hundert bis größer als 100000 betragen. Die Substanzen können neutral oder elektrisch geladen sein. Bevorzugt sind Salze saurer Farbstoffe mit physiologisch verträglichen Basen, wie Natrium, Methylglutamin, Lysin oder Salze mit Lithium, Calcium, Magnesium, Gadolinium als Kationen.

Gegenstand der vorliegenden Erfindung ist die Verwendung von Cyaninfarbstoffen der allgemeinen Formel V zur Herstellung von Mitteln zur In-vitro-Diagnostik mittels NIR-Strahlung.

Ein Gegenstand der vorliegenden Erfindung sind ferner auch diagnostische Mittel, welche Verbindungen der allgemeinen Formel V enthalten.

Diese Mittel werden nach den dem Fachmann bekannten Methoden hergestellt, ggf. unter Verwendung üblicher Hilfs-und/oder Trägerstoffe sowie Verdünnungsmittel und dergleichen. Dazu gehören physiologisch verträgliche Elektrolyte, Puffer, Detergenzien und Substanzen zur Anpassung der Osmolalität sowie zur Verbesserung der Stabilität und Löslichkeit, wie beispielsweise Cyclodextrine. Durch die in der Pharmazie gebräuchlichen Maßnahmen ist für die Sterilität der Zubereitungen bei der Herstellung und insbesondere vor der Applikation zu sorgen.

Die nachfolgenden Beispiele erläutern die Erfindung.

### Beispiel 1:

Darstellung von 5-[2-[(1,2-Dicarboxyethyl)amino]-2-oxoethyl]-2-[7-[5-[2-[(1,2-dicarboxyethyl)amino]-2-oxoethyl]-1,3-dihydro-3,3-dimethyl-1-(4-sulfobutyl)-2*H*-indol-2-yliden]-1,3,5-heptatrienyl]-3,3-dimethyl-1-(4-sulfobutyl)-3*H*-indolium, inneres Salz, Monokaliumsalz

Aus 5-Carboxymethyl-2-[7-[5-carboxymethyl-1,3-dihydro-3,3-dimethyl-1-(4-sulfobutyl)-2*H*-indol-2-yliden]-1,3,5-heptatrienyl]-3,3-dimethyl-1-(4-sulfobutyl)-3*H*-indolium, inneres Salz, Monokaliumsalz wird nach bekannten Verfahren der Di-N-Hydroxysuccinimid-Ester dargestellt (Cytometry 11 (1990) 418-430).
Zu einer Lösung von 0,5 g (0,51 mmol) des Disuccinimidylesters in 5 ml DMF werden 0,16 g (1,22 mmol) Asparaginsäure in 1 ml DMF gegeben. Das Reaktionsgemisch wird 48 h bei Raumtemp. gerührt und das Produkt durch Zugabe von Ether ausgefällt. Reinigung an RP-18 (LiChroprep, 15-25µ, H₂O:MeOH 99:1 bis 1:1) und anschließende Gefriertrocknung, sowie 24 stdg. Trocknung bei 50°C/0,01 mbar ergibt 0,27 g (51%) Produkt.

| Analyse: | | | | | | |
|---|---|---|---|---|---|---|
| Ber. | C 54,43 | H 5,54 | N 5,40 | O 24,68 | S 6,18 | K 3,77 |
| Gef. | C 54,04 | H 5,81 | N 5,22 | | S 6,13 | K 3,85 |

### Beispiel 2:

Darstellung von 2-[7-[5-[2-[(11-Carboxy-2-oxo-1,4,7,10-tetraaza-4,7,10-tri(carboxymethyl)-1-undecyl)amino]-2-oxoethyl]-1,3-dihydro-3,3-dimethyl-1-ethyl-2*H*-indol-2-yliden]-1,3,5-heptatrienyl]-3,3-dimethyl-1-(4-sulfobutyl)-3*H*-indolium, inneres Salz.

Zu einer Lösung von 0,5 g (0,73 mmol) 2-[7-[5-(Carboxymethyl)-1,3-dihydro-3,3-dimethyl-1-ethyl-2*H*indol-2-yliden]-1,3,5-hepta-trienyl]-3,3-dimethyl-1-(4-sulfobutyl)-3*H*-indolium-N-succinimidylester, inneres Salz(Cytometry 11 (1990) 418-430) in 5 ml Methanol werden 43 mg (0,65 mmol) 85-proz. Hydrazin-hydrat in 1 ml Methanol langsam bei -10 °C zugetropft und 2 h bei dieser Temp. gerührt. Die Reaktionslösung wird unter Vakkuum auf ca. 3 ml eingedampt, mit 1 ml Isopropanol versetzt und über Nacht bei -20°C aufbewahrt. Die ausgefallenen Kristalle werden abgesaugt und an der Ölpumpe getrocknet. Man erhält 0,27 g (61%) Tricarbocyanincarbonsäurehydrazid.
Zu einer Lösung von 0,21 g (0,51 mmol) Diethylentriaminpentaessigsäuremonoethylestermonoanhydrid in 20 ml DMF und 0,2 ml Triethylamin werden unter Rühren 0,27 g (0,45 mmol)des Hydrazids gegeben, das Gemisch wird 48 h bei Raumtemp. gerührt. Nach Filtration wird das Lösungsmittel bei 0,2 mbar verdampft, der Rückstand mit CH₂Cl₂ verrührt, abfiltriert und im Hochvakuum getrocknet. Das erhaltene Produkt wird in 5 ml 3M wäßriger NaOH 4 h bei Raumtemp. gerührt, dann wird mit halbkonz. HCl auf pH 2,0 eingestellt, 1 ml Isopropanol zugefügt und die nach 18-stdg. Stehen bei 4°C erhaltenen Kristalle abgesaugt und im Hochvakuum 24 h bei 60 °C getrocknet, Ausbeute 0,23 g (52%)als dunkles, rot schimmerndes Granulat.

| | | | | | | |
|---|---|---|---|---|---|---|
| Analyse | Ber. | C 59,32 | H 6,60 | N 9,88 | O 20,96 | S 3,23 |
| | Gef. | C 54,15 | H 6,70 | N 9,50 | | S 3,19 |

### Beispiel 3:

Darstellung von 2-[7-[1,3-Dihydro-3,3-dimethyl-5-[2-[(Methoxypolyoxyethylen)-amino]-2-oxoethyl]-1-(4-sulfobutyl)-2H-indol-2-yliden]-1,3,5-heptatrienyl]-3,3-dimethyl-5-[2-[(Methoxypolyoxyethylen)amino]-2-oxoethyl]-1-(4-sulfobutyl)-3H-indolium, Natriumsalz

Zu einer Lösung von 800 mg Methoxypolyoxyethylenamin (ca. 0,16 mmol; mittlere Molmasse ca. 5000) in 10 ml CH₂Cl₂ gibt man eine Lösung von 0,08 mmol des N,N-Disuccinimidyltesters aus Beispiel 1 in 1 ml DMF und läßt 24 h bei Raumtemperatur rühren. Der nach Zugabe von Ether ausgefallene Feststoff wird abfiltriert und chromatographisch gereinigt (Sephadex G50 medium, H₂O als Eluens), Ausbeute ca. 58% als grünblaues Pulver nach Gefriertrocknung und Trocknung über P₂O₅.
mittlere Molmasse ber.: 10771, gef.: 10820

### Beispiel 4 :

2-[7-[1,3-Dihydro-3,3-dimethyl-1-(4-sulfobutyl)-2H-indol-2-yliden]-1,3,5-heptatrienyl]-3,3-dimethyl-5-(methoxypolyoxyethylen)aminocarbonyl-1-(4-sulfobutyl)-3Hindolium, Natriumsalz

0,41 g (0,5 mmol) 2-[7[1,3-Dihydro-3,3-dimethyl-1-(4-sulfobutyl-1-(4-sulfobutyl)-2H-indol-2-yliden]-1,3,5-hepatrienyl]-3,3-dimethyl-5-carboxy-1-(4-sulfobutyl)-3Hindolium-N-succinimidyl ester, Natriumsalz wird zusammen mit 2,3 g Methoxypolyoxyethylenamin (0,46 mmol; mittlere Molmasse 5000) 18 h in 70 ml CH₂Cl₂ bei Raumtemperatur unter Argon gerührt, das Lösungsmittel im Vakuum auf die Hälfte eingeengt und das Produkt wie in Beispiel 3 beschrieben isoliert. Man erhält 2,1 g Produkt als grünblaues Pulver.
mittlere Molmasse ber.: 5701, gef.: 5795

### Beispiel 5:

Darstellung von 3-(3-Carboxypropyl)-2-[7-[3-(3-carboxypropyl)-1,3-dihydro-3-methyl-1-(4-sulfobutyl)-2H-indol-2-yliden]-1,3,5-heptatrienyl]-3-methyl-1-(4-sulfobutyl)-3*H*indolium, Natriumsalz.

6,5 g (50 mmol) Phenylhydrazin-hydrochlorid und 8,7 g (55 mmol) 5-Methyl-6-Oxoheptansäure werden 50 ml konz. Essigsäure 1 h bei Raumtemp. und 5 h bei 120°C gerührt. Nach Einengen wird der Rückstand mit 20 ml Wasser verrührt und die entstandenen Kristalle abfiltriert und an der Ölpumpe getrocknet.
Man erhält 9,6 g (83%) bräunliche Kristalle, welche in 60 ml Dichlorbenzol suspendiert und nach Zugabe von 11,6 g (85 mmol) 1,4-Butansulton 8 h auf 150°C erhitzt werden. Nach Abkühlen auf Raumtemp. werden 200 ml Aceton zugegeben und der entstandene Niederschlag abfiltriert. Dieser wird in Ether suspendiert und nach 18-stdg. Rühren erneut abfiltriert und an der Ölpumpe getrocknet. Man erhält 10,7 g (70%) 3-(3-Carboxypropyl)-2,3-dimethyl-1-(4-Sulfobutyl)-3*H*-indolenin, welches chromatographisch gereinigt wird (RP-18, LiChroprep, 15-25 µ, MeOH:H₂O als Eluens).

Die Darstellung des Indotricarbocyaninfarbstoffs erfolgt durch 30 min. erhitzen von 5,0 g (13,6 mmol) Indolenin und 1,9 g (6,8 mmol) Glutaconaldehyddianilhydrochlorid in 100 ml Essigsäureanhydrid unter Zusatz von 25 ml konz. Essigsäure und 2,3 g (27,6 mmol) wasserfreiem Natriumacetat auf 120°C. Der nach Zusatz von 500 ml Ether erhaltene Niederschlag wird chromatographisch gereinigt (in 1,0 g-Portionen, RP-18, LiChroprep, 15-25 µ, MeOH:H₂O als Eluens) und abschließend gefriergetrocknet. Man erhält 2,5 g (45%) Produkt.

| Analyse: | | | | | | |
|---|---|---|---|---|---|---|
| Ber. | C 60,13 | H 6,28 | N 3,42 | O 19,54 | S 7,83 | Na 2,81 |
| Gef. | C 59,90 | H 6,34 | N 3,39 | | S 7,72 | Na 2,78 |

### Beispiel 6:

Darstellung von 2-[7-[1,3-Dihydro-5-[2-[(2,3-dihydroxypropyl)amino]-2-oxoethyl]-3,3-dimethyl-1-(4-sulfobutyl)-2H-indol-2-yliden]-1,3,5-heptatrienyl]-5-[2-[(2,3-dihydroxypropyl)amino]-2-oxoethyl]-3,3-dimethyl-1-(4-sulfobutyl)-3H-indolium, Natriumsalz 2,0 g (6,4 mmol) 2,3,3-Trimethyl-3H-indol-5-ylessigsäuresuccinimidyl-ester werden in 50 ml CH₂Cl₂ mit 0,84 g (6,4 mmol) 4-Aminomethyl-2,2-dimethyl-1,3-dioxolan versetzt. Nach 5 stdg. Rühren bei Raumtemp. wird auf 100 ml Wasser gegossen, mit CH₂Cl₂ extrahiert und die org. Phasen eingeengt. Nach chromatographischer Reinigung (Kieselgel CH₂Cl₂ : MeOH 98:2) erhält man 1,86 g (88%) des Amids, welches in 20 ml Dichlorbenzol mit 1,36 g (10,0 mmol) 1,4-Butansulton 7 h bei 100°C und 12 h bei Raumtemp. gerührt wird. Das nach Verrühren mit 50 ml Aceton entstandene Granulat wird abfiltriert und chromatographisch gereinigt (RP-18, LiChroprep, 15-25 µ, MeOH:H₂O als Eluens). Man erhält 0,85 g (28% bezogen auf die Ausgangsverbindung) 5-[2-[(2,2-Dimethyl-1,3-dioxa-4-cyclopentyl)methyl]amino-2-oxoethyl]-2,3,3-trimethyl-1-(4-sulfobutyl-3*H*-indolenin.

Die Umsetzung zum Farbstoff erfolgt in Anlehnung an Beispiel 4, indem 10 min. auf 120 °C erhitzt wird. Das Rohprodukt wird in 5 ml MeOH unter Zusatz von 100 mg p-Toluolsulfonsäure 16 h bei Raumtemp. gerührt, unlösliche Anteile werden abgetrennt und das Filtrat anschließend nach Zusatz von 3 ml Isopropanol bei -20°C aufbewahrt.
Das ausgefallene Pulver wird chromatographisch gereinigt (RP-18, LiChroprep, 15-25 µ, MeOH:H₂O als Eluens), gefriergetrocknet und 24 h bei 50°C/0,01 mbar getrocknet, Ausbeute 0,32 g (37%).

| Analyse: | | | | | | |
|---|---|---|---|---|---|---|
| Ber. | C 56,70 | H 6,45 | N 5,88 | O 20,14 | S 6,73 | K 4,10 |
| Gef. | C 56,39 | H 6,88 | N 5,67 | | S 6,58 | K 3,93 |

### Beispiel 7 :

Einer narkotisierten, tumortragenden Nacktmaus (Swiss-Nude, Tumor LS 174 T an der rechten Hinterflanke) wurde 2-[7-[1,3-Dihydro-3,3-dimethyl-5-(methoxycarbonyl)-1-(4-sulfobutyl)-2*H*-indol-2-yliden]-1,3,5-heptatrienyl]-3,3-dimethyl-5-(methoxycarbonyl)-1-(4-sulfobutyl)-3*H*indolium, Natriumsalz in einer Dosis von 3,8 µmol/kg Körpergewicht i.v. appliziert.

Die laserinduzierten Fluoreszenzaufnahmen wurden vor und zu verschiedenen Zeiten nach Substanzapplikation mit einem Fluoreszenz-Imager (Physikalisch-Technische Bundesanstalt, Berlin Charlottenburg) durchgeführt. Die Anregung erfolgte mit monochromatischem Laserlicht bei 740 nm durch Auskopplung der Strahlung über ein Lichtleitersystem. Anregerstrahlung unterhalb 740 nm wurde durch einen Kantenfilter entfernt und das laserinduzierte Fluoreszenzlicht oberhalb 740 nm mit einer CCD-Kamera (Charge Coupled Device)aufgenommen und die Daten als Schwarzweiß-Bilder gespeichert.

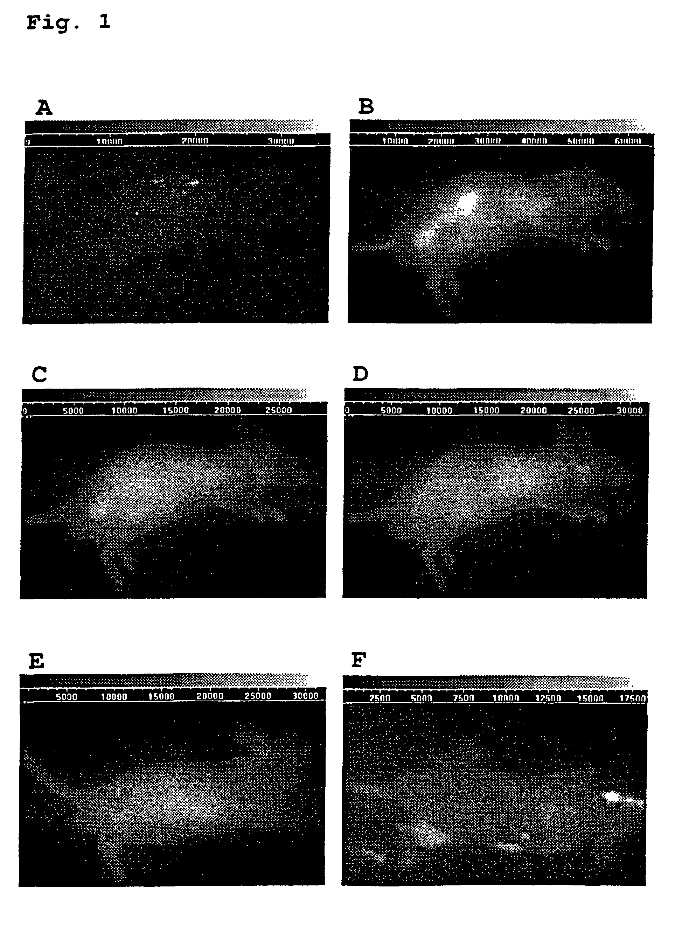

Die Aufnahmesequenz der Fig. 1 zeigt deutlich die allgemeine Zunahme der Fluoreszenzintensität nach Substanzapplikation (Bild A, B). Nach 30 sek. ist eine gleichverteilte Intensität mit erhöhten Werten im Leber-Lunge-Bereich und Tumor zu beobachten (B). Im weiteren Zeitverlauf bis zu 1 h (C,D,E) verteilt sich die Substanz zunehmend im Tier. Nach 18 h ist im Tumor (rechte Hinterflanke) eine gegenüber dem Restkörper deutlich erhöhte Fluoreszenzintensität zu beobachten.

Die Figur 1 zeigt Fluoreszenzlichtaufnahmen (Schwarzweiß-Bild) einer Nacktmaus (Swiss-Nude) zu verschiedenen Zeitpunkten nach i.v. Applikation von 3,8 µmol/kg Körpergewicht 2-[7-[1,3-Dihydro-3,3-dimethyl-5-(methoxycarbonyl)-1-(4-sulfobutyl)-2*H*-indol-2-yliden]-1,3,5-heptatrienyl]-3,3-dimethyl-5-(methoxycarbonyl)-1-(4-sulfobutyl)-3*H*-indolium, Natriumsalz
A-E: rechtslaterale Aufnahmen, F: posteriore Aufnahme
A: vor Applikation,
B: 30 sek.,
C: 1 min,
D: 10 min,
E: 1 h nach Applikation,
F: 18 h nach Applikation.

## Patentansprüche

1. Cyaninfarbstoffe der allgemeinen Formel V 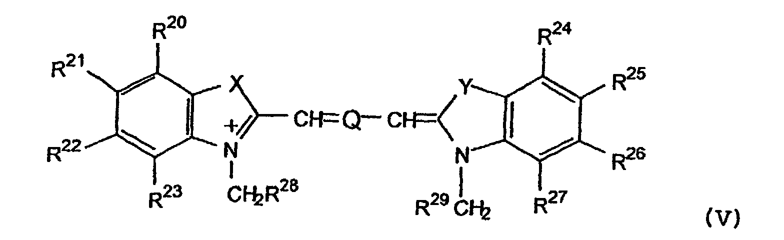 wobei
Q ein Fragment 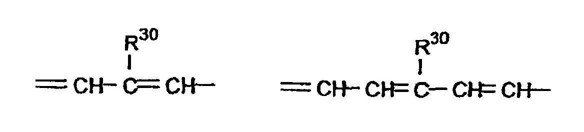 oder 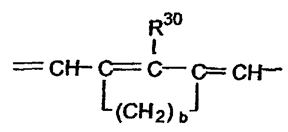 ist,
wobei R³⁰ für ein Wasserstoffatom, eine Hydroxygruppe, eine Carboxygruppe, einen Alkoxyrest mit 1 bis 4 Kohlenstoffatomen oder ein Chloratom steht, b eine ganze Zahl 2 oder 3 bedeutet,
X und Y unabhängig voneinander für ein Fragment -O-, -S-, -CH=CH- oder -(CH₂R³²)(CH₂R³³)- stehen,
R²⁰ bis R²⁹, R³² und R³³ unabhängig voneinander für ein Wasserstoffatom, eine Hydroxygruppe, einen Carboxy-, einen Sulfonsäure-Rest oder einen Carboxyalkyl-, Alkoxycarbonyl-, oder Alkoxyoxoalkyl-Rest mit bis zu 10 C-Atomen oder ein Sulfoalkylrest mit bis zu 4 C-Atomen,
oder für ein Polylysin, Polyethylenglycol, Methoxypolyethylenglycol, Polyvinylalkohol, Dextran, Carboxydextran oder eine kaskadenpolymerartige Struktur,
oder für einen Rest der allgemeinen Formel VI
**-(O)**_{**v**}**-(CH**_{**2**}**)**_{**o**}**-CO-NR**^{**34**}**-(CR**_{**2**}**)**_{**s**}**-(NH-CO)**_{**q**}**-R**^{**35**} (VI)
steht,
mit der Maßgabe, daß bei der Bedeutung von X und Y gleich O, S, -CH=CH- oder -C(CH₃)₂mindestens einer der Reste R²⁰ bis R²⁹ einem Polylysin, Polyethylenglycol, Methoxypolyethylenglycol, Polyvinylalkohol, Dextran, Carboxydextran oder einer kaskadenpolymerartigen Struktur oder der allgemeinen Formel VI entspricht,
wobei
o und s gleich 0 sind oder unabhängig voneinander für eine ganze Zahl von 1 bis 6 stehen,
q und v unabhängig voneinander für 0 oder 1 stehen,
R³⁴ ein Wasserstoffatom oder einen Methylrest darstellt,
R³⁵ ein Alkylrest mit 3 bis 6 C-Atomen, welcher 2 bis n-1 Hydroxygruppen aufweist, wobei n die Anzahl der C-Atome ist, oder ein mit 1 bis 3 Carboxygruppen substituierter Alkylrest mit 1 bis 6 C-Atomen, Arylrest mit 6 bis 9 C-Atomen oder Arylalkylrest mit 7 bis 15 C-Atomen, oder ein Rest der allgemeinen Formel IIId oder IIIe 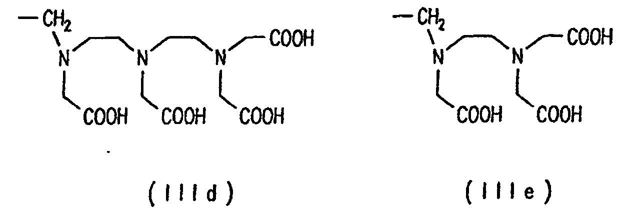 ist,
unter der Maßgabe, daß q für 1 steht,
oder ein Polylysin, Polyethylenglycol, Methoxypolyethylenglycol, Polyvinylalkohol, Dextran, Carboxydextran oder eine kaskadenpolymerartige Struktur bedeutet, oder
R²⁰ und R²¹, R²¹ und R²², R²² und R²³, R²⁴ und R²⁵, R²⁵ und R²⁶, oder R²⁶ und R²⁷ zusammen mit den zwischen ihnen liegenden Kohlenstoffatomen einen 5- oder 6-gliedrigen aromatischen oder gesättigten annelierten Ring bilden,
sowie deren physiologisch verträgliche Salze,
wobei die Cyaninfarbstoffe eine durch einen n-Octanol/Wasser-Verteilungskoeffizienten von kleiner 2,0 gekennzeichnete Hydrophilie besitzen.

2. Cyaninfarbstoff nach Anspruch 1, nämlich
5-[2-[(1,2-Dicarboxyethyl)amino]-2-oxoethyl]-2-[7-[5-[2-[(1,2-dicarboxyethyl)amino]-2-oxoethyl]-1,3-dihydro-3,3-dimethyl-1-(4-sulfobutyl)-2*H*-indol-2-yliden]-1,3,5-heptatrienyl]-3,3-dimethyl-1-(4-sulfobutyl)-3*H*-indolium, inneres Salz, Monokaliumsalz,
2-[7-[5-[2-[(11-Carboxy-2-oxo-1,4,7,10-tetraaza-4,7,10-tri(carboxymethyl)-1-undecyl)amino]-2-oxoethyl]-1,3-dihydro-3,3-dimethyl-1-ethyl-2*H*-indol-2-yliden]-1,3,5-heptatrienyl]-3,3-dimethyl-1-(4-sulfobutyl)-3*H*-indolium, inneres Salz,
2-[7-[1,3-Dihydro-3,3-dimethyl-5-[2-[(Methoxypolyoxyethylen)-amino]-2-oxoethyl]-1-(4-sulfobutyl)-2Hindol-2-yliden]-1,3,5-heptatrienyl]-3,3-dimethyl-5-[2-[(methoxypolyoxyethylen)amino]-2-oxoethyl]-1-(4-sulfobutyl)-3H-indolium, Natriumsalz,
2-[7-[1,3-Dihydro-3,3-dimethyl-1-(4-sulfobutyl)-2Hindol-2-yliden]-1,3,5-heptatrienyl]-3,3-dimethyl-5-(Methoxypolyoxyethylen)aminocarbonyl-1-(4-sulfobutyl)-3H-indolium, Natriumsalz
3- (3-Carboxypropyl)-2-[7-[3-(3-carboxypropyl)-1,3-dihydro-3-methyl-1-(4-sulfobutyl)-2*H*-indol-2-yliden]-1,3,5-heptatrienyl]-3-methyl-1-(4-sulfobutyl)-3*H*indolium, Natriumsalz,
2-[7-[1,3-Dihydro-5-[2-[(2,3-dihydroxypropyl)amino]-2-oxoethyl] -3,3-dimethyl-1-(4-sulfobutyl) -2H-indol-2-yliden]-1,3,5-heptatrienyl]-5-[2-[(2,3-dihydroxypropyl)amino]-2-oxoethyl]-3,3-dimethyl-1-(4-sulfobutyl)-3H-indolium, Natriumsalz

3. Verwendung von Cyaninfarbstoffen nach Anspruch 1 oder 2 zur Herstellung von Mitteln für die In-vivo-Diagnostik mittels NIR-Strahlung.

4. Mittel zur In-vivo-Diagnostik, **dadurch gekennzeichnet, daß** es mindestens einen der Cyaninfarbstoffe nach Anspruch 1 oder 2 zusammen mit den üblichen Hilfs- und Trägerstoffen sowie Verdünnungsmitteln enthält.

## Claims

1. Cyanine dyes of the general formula V 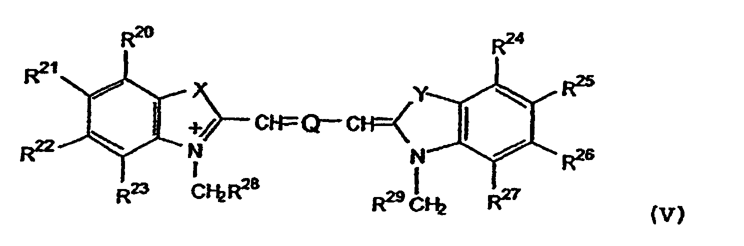 where Q represents a fragment 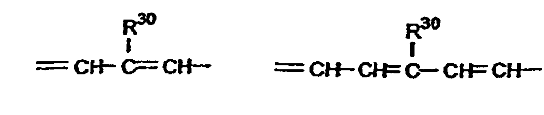 or 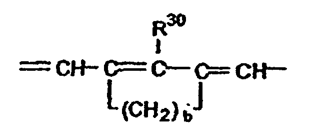 where R³⁰ represents a hydrogen atom, a hydroxy group, a carboxy group, an alkoxy residue containing 1 to 4 carbon atoms or a chlorine atom,
b is an integer (2 or 3),
X and Y independently represent an -O-, -S-, -CH=CH- or -C(CH₂R³²) (CH₂R³³) - fragment each,
R²⁰ to R²⁹, R³² and R³³ independently represent a hydrogen atom, a hydroxy group, a carboxy, a sulphonic acid residue or a carboxyalkyl, alkoxycarbonyl or alkoxyoxoalkyl residue containing up to 10 C atoms or a sulphoalkyl residue containing up to 4 C atoms
or represents a polylysine, polyethylene glycol, methoxypolyethylene glycol, polyvinyl alcohol, dextran, carboxydextran or a cascade polymer-like structure, or represents a residue of the general formula VI
- (O)ᵥ-(CH₂)ₒ-CO-NR³⁴-(CH₂)ₛ-(NH-CO)_{q}-R³⁵ (VI)
on the condition that, where X and Y are O, S, -CH=CH- or -C(CH₃)₂-, at least one of the residues R²⁰ to R²⁹ corresponds to a polylysine, polyethylene glycol, methoxypolyethylene glycol, polyvinyl alcohol, dextran, carboxydextran or a cascade polymer-like structure, or a residue of the general formula VI,
where
o and s equal 0 or independently represent an integer between 1 and 6,
q and v independently represent 0 or 1,
R³⁴ represents a hydrogen atom or a methyl residue,
R³⁵ represents an alkyl residue containing 3 to 6 C atoms and comprising 2 to n-1 hydroxy groups, with n being the number of C atoms, or an alkyl residue containing 1 to 6 C atoms that is substituted by 1 to 3 carboxy groups, an aryl residue containing 6 to 9 C atoms or arylalkyl residue containing 7 to 15 C atoms, or a residue of the general formula IIId or IIIe 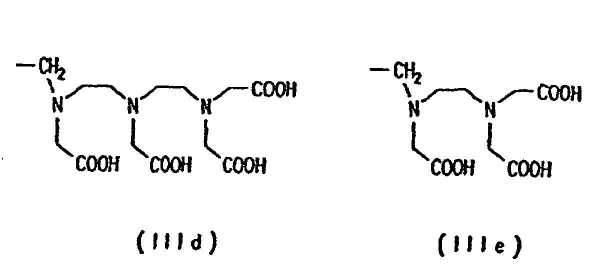 on the condition that q is 1, or a polylysine, polyethylene glycol, methoxypolyethylene glycol, polyvinyl alcohol, dextran, carboxydextran or a cascade polymer-like structure, or
R²⁰ and R²¹, R²¹ and R²², R²² and R²³, R²⁴ and R²⁵, R²⁵ and R²⁶ or R²⁶ and R²⁷, together with the interspersed carbon atoms, form a 5- or 6-member aromatic or saturated fused ring,
as well as their physiologically tolerable salts,
the cyanine dyes having a hydrophilicity **characterized by** an n-octanol/water distribution coefficient of less than 2.0.

2. Cyanine dye according to Claim 1, namely
5-[2-[(1,2-dicarboxyethyl)amino]-2-oxoethyl]-2-[7-[5-[2-[(1,2-dicarboxyethyl)amino]-2-oxoethyl]-1,3-dihydro-3,3-dimethyl-1-(4-sulphobutyl)-2H-indol-2-ylidene]-1,3,5-heptatrienyl]-3,3-dimethyl-1-(4-sulphobutyl)-3H-indolium, inner salt, potassium hydrogen salt;
2-[7-[5-[2-[(11-carboxy-2-oxo-1,4,7,10-tetraaza-4,7,10-tri(carboxymethyl)-1-undecyl)amino]-2-oxoethyl]-1,3-dihydro-3,3-dimethyl-1-ethyl-2H-indol-2-ylidene]-1,3,5-heptatrienyl]-3,3-dimethyl-1-(4-sulphobutyl)-3H-indolium, inner salt;
2-[7-[1,3-dihydro-3,3-dimethyl-5-[2-[(methoxypoly-oxyethylene)-amino]-2-oxoethyl]-1-(4-sulphobutyl)-2H-indol-2-ylidene]-1,3,5-heptatrienyl]-3,3-dimethyl-5-[2-[(methoxypolyoxyethylene)amino]-2-oxoethyl]-1-(4-sulphobutyl)-3H-indolium, sodium salt;
2-[7-[1,3-dihydro-3,3-dimethyl-1-(4-sulphobutyl)-2H-indol-2-ylidene]-1,3,5-heptatrienyl]-3,3-dimethyl-5-(methoxypolyoxyethylene)aminocarbonyl-1-(4-sulphobutyl)-3H-indolium, sodium salt;
3-(3-carboxypropyl)-2-[7-[3-(3-carboxypropyl)-1,3-dihydro-3-methyl-1-(4-sulphobutyl)-2H-indol-2-ylidene]-1,3,5-heptatrienyl]-3-methyl-1-(4-sulphobutyl)-3 H-indolium, sodium salt;
2-[7-[1,3-dihydro-5-[2-[(2,3-dihydroxypropyl)amino]-2-oxoethyl]-3,3-dimethyl-1-(4-sulphobutyl)-2H-indol-2-ylidene]-1,3,5-heptatrienyl]-5-[2-[(2,3-dihydroxypropyl)amino]-2-oxoethyl]-3,3-dimethyl-1-(4-sulphobutyl)-3H-indolium. sodium salt.

3. Use of cyanine dyes according to claim 1 or 2 for preparing agents for in vivo diagnostics by means of NIR radiation.

4. Agent for in vivo diagnostics, **characterized in that** it contains at least one of the cyanine dyes according to claim 1 or 2 together with the customary adjuvant and excipient substances and also diluents.

## Revendications

1. Colorants cyanines de formule générale V 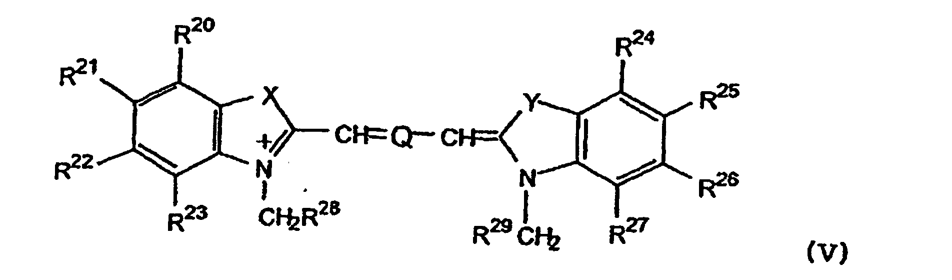 dans laquelle
Q représente un fragment 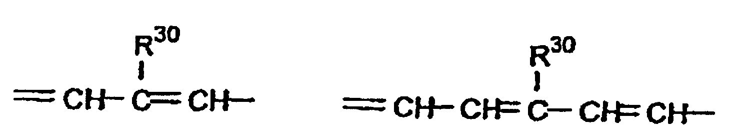 ou 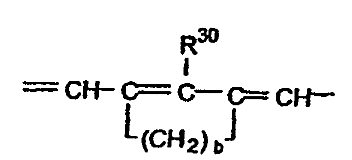 où R³⁰ représente un atome d'hydrogène, un groupe hydroxy, un groupe carboxy, un radical alcoxy ayant de 1 à 4 atomes de carbone ou un atome de chlore, b représente un entier 2 ou 3,
X et Y représentent, indépendamment l'un de l'autre, un fragment -O-, -S-, -CH=CH- ou -C(CH₂R³²) (CH₂R³³)-,
R²⁰ à R²⁹, R³² et R³³ représentent, indépendamment les uns des autres, un atome d'hydrogène, un groupe hydroxy, un radical carboxy ou acide sulfonique, ou un radical carboxyalkyle, alcoxycarbonyle ou alcoxyoxoalkyle ayant jusqu'à 10 atomes de carbone ou un radical sulfoalkyle ayant jusqu'à 4 atomes de carbone,
ou une polylysine, un polyéthylèneglycol, un méthoxypolyéthylèneglycol, un alcool polyvinylique, un dextrane, un carboxydextrane ou une structure du type polymère en cascade,
ou un radical de formule générale VI
- **(O)**_{**v**}**-(CH**_{**2**}**)**_{**o**}**-CO-NR**^{**34**}**-(CH**_{**2**}**)**_{**s**}**-(NH-CO)**_{**q**}**-R**^{**35**} (VI)
à condition que lorsque X et Y représentent O, S, -CH=CH- ou -C(CH₃)₂-, au moins l'un des radicaux R²⁰ à R²⁹ représente une polylysine, un polyéthylèneglycol, un méthoxypolyéthylèneglycol, un alcool polyvinylique, un dextrane, un carboxydextrane ou une structure du type polymère en cascade ou correspond à la formule générale VI,
où
o et s valent 0 ou représentent, indépendamment l'un de l'autre, un entier de 1 à 6,
q et v valent, indépendamment l'un de l'autre, 0 ou 1,
R³⁴ représente un atome d'hydrogène ou un radical méthyle,
R³⁵ représente un radical alkyle ayant de 3 à 6 atomes de carbone renfermant de 2 à n-1 groupes hydroxy, où n est le nombre d'atomes de carbone, ou un radical alkyle ayant de 1 à 6 atomes de carbone, substitué par de 1 à 3 groupes carboxy, un radical aryle ayant de 6 à 9 atomes de carbone ou un radical arylalkyle ayant de 7 à 15 atomes de carbone, ou un radical de formule générale IIId ou IIIe 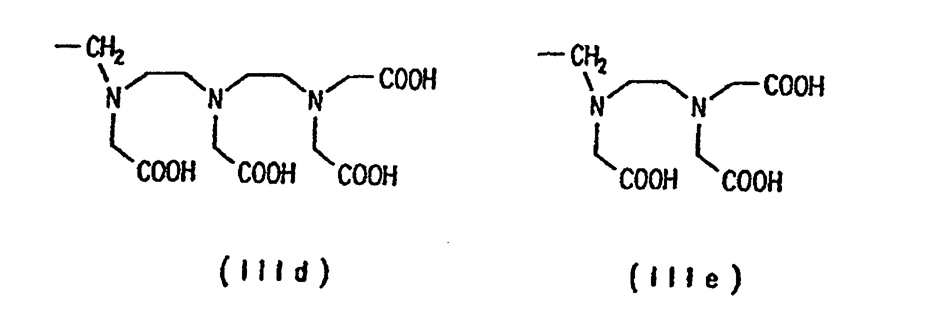 à condition que q vale 1,
ou représente une polylysine, un polyéthylèneglycol, un méthoxypolyéthylèneglycol, un alcool polyvinylique, un dextrane, un carboxydextrane ou une structure du type polymère en cascade, ou
R²⁰ et R²¹, R²¹ et R²², R²² et R²³, R²⁴ et R²⁵, R²⁵ et R²⁶, ou R²⁶ et R²⁷ forment, conjointement avec les atomes de carbone se trouvant entre eux, un noyau condensé aromatique ou saturé à 5 ou 6 chaînons,
ainsi que leurs sels physiologiquement acceptables,
les colorants cyanines présentant une hydrophilie **caractérisée par** un coefficient de partage n-octanol/eau inférieur à 2,0.

2. Colorant cyanine selon la revendication 1, à savoir
le 5-[2-[(1,2-dicarboxyéthyl)amino]-2-oxoéthyl]-2-[7-[5-[2-[(1,2-dicarboxyéthyl)amino]-2-oxoéthyl]-1,3-dihydro-3,3-diméthyl-1-(4-sulfobutyl)-2*H*-indol-2-ylidène]-1,3,5-heptatriényl]-3,3-diméthyl-1-(4-sulfobutyl)-3*H*-indolium, sel interne, sel monopotassique,
le 2-[7-[5-[2-[(11-carboxy-2-oxo-1,4,7,10-tétraaza-4,7,10-tri(carboxyméthyl)-1-undécyl)amino]-2-oxoéthyl]-1,3-dihydro-3,3-diméthyl-1-éthyl-2*H*-indol-2-ylidène]-1,3,5-heptatriényl]-3,3-diméthyl-1-(4-sulfobutyl)-3*H*-indolium, sel interne,
le 2-[7-[1,3-dihydro-3,3-diméthyl-5-[2-[(méthoxypoly-oxyéthylène)-amino]-2-oxoéthyl]-1-(4-sulfobutyl)-2H-indol-2-ylidène]-1,3,5-heptatriényl]-3,3-diméthyl-5-[2-[(méthoxypolyoxyéthylène)amino]-2-oxoéthyl]-1-(4-sulfobutyl)-3H-indolium, sel sodique,
le 2-[7-[1,3-dihydro-3,3-diméthyl-1-(4-sulfobutyl)-2H-indol-2-ylidène]-1,3,5-heptatriényl]-3,3-diméthyl-5-(méthoxypolyéthylène)aminocarbonyl-1-(4-sulfobutyl)-3H-indolium, sel sodique,
le 3-(3-carboxypropyl)-2-[7-[3-(3-carboxypropyl)-1,3-dihydro-3-méthyl-1-(4-sulfobutyl)-2*H*-indol-2-ylidène]-1,3,5-heptatriényl]-3-méthyl-1-(4-sulfobutyl)-3*H*-indolium, sel sodique,
le 2-[7-[1,3-dihydro-5-[2-[(2,3-dihydroxypropyl)amino]-2-oxoéthyl]-3,3-diméthyl-1-(4-sulfobutyl)-2H-indol-2-ylidène]-1,3,5-heptatriényl]-5-[2-[(2,3-dihydroxypropyl)amino]-2-oxoéthyl]-3,3-diméthyl-1-(4-sulfobutyl)-3H-indolium, sel sodique.

3. Utilisation de colorants cyanines selon la revendication 1 ou 2, pour la préparation de compositions destinées au diagnostic in vivo au moyen d'un rayonnement proche infrarouge.

4. Composition destinée au diagnostic in vivo, **caractérisée en ce qu'**elle comprend au moins l'un des colorants cyanines selon la revendication 1 ou 2 conjointement avec les auxiliaires et supports ainsi que les diluants habituels.
